# EUROPÄISCHE PATENTANMELDUNG

(11) **EP 2 727 628 A2**
(43) Veröffentlichungstag der Anmeldung: **07.05.2014**
(21) Anmeldenummer: 13187365.5
(22) Anmeldetag: 04.10.2013
(51) Int. Cl.: A61Q 19/10, A61Q 19/00, A61K 8/06, A61K 8/81

(54) **Pflegendes, kosmetisches Reinigungsmittel**

(30) Priorität: 05.11.2012 DE 102012220077
(71) Anmelder: Henkel AG & Co. KGaA, 40589 Düsseldorf (DE)
(72) Erfinder: Sartingen, Kirsten, 41379 Brüggen (DE); Heide, Barbara, 47809 Krefeld (DE)

(57) **Zusammenfassung**

Kosmetisches Reinigungsmittel mit einer Viskosität (Haake Rotationsviskosimeter RV 12, 20°C, Meßeinrichtung MV; Spindel MV II; 8UPM) von 5000 bis 14000 mPas, enthaltend
a) mindestens ein anionisches Tensid
b) mindestens ein amphoteres Tensid
c) mindestens einen Fettstoff
d) mindestens ein anionisches Copolymer aus den Monomeren
d1) Ethylacrylat
d2) Butylacrylat
d3) Methacrylsäure
sowie gegebenenfalls weiteren Monomeren.
zeichnen sich durch eine gute kosmetische Eigenschaften und eine hohe physikalische Stabilität aus.

## Beschreibung

Die Anmeldung Erfindung liegt auf dem Gebiet der Kosmetik und betrifft ein kosmetisches Reinigungsmittel, das neben Tensiden und pflegenden Wirkstoffen mindestens ein spezifisches anionisches Copolymer enthält.

Kosmetische Reinigungsmittel sind lange bekannt und werden regelmäßig verbessert bzw. den wechselnden Bedürfnissen der Verbraucher angepasst. Beispielsweise erwarten Verbraucher von einem modernen Reinigungsmittel nicht nur eine gute Reinigung und Erfrischung, sondern auch pflegende Eigenschaften. Insbesondere sollen Reinigungsmittel nach ihrer Anwendung auf der Haut kein Spannungsgefühl und/oder keine Trockenheit hinterlassen. Die Haut soll sich nach der Reinigung vielmehr weich, glatt und befeuchtet anfühlen. Zudem spielt auch der optische und haptische Eindruck eines Reinigungsmittels eine zunehmend wichtige Rolle, weshalb die Herstellung von Produkten mit reichhaltiger, cremiger Konsistenz besonders erstrebenswert ist.

Es ist bekannt, kosmetischen Reinigungsmitteln zur Verbesserung der Hautpflege pflegende Öle und/oder Wachse hinzuzufügen. Die Herstellung solcher pflegender Reinigungsmittel ist jedoch problematisch, denn zum einen bereitet die Stabilisierung höherer Öl- und/oder Wachsmengen (die zur Erzielung eines ausreichenden Pflegeeffektes erstrebenswert sind) oftmals erhebliche Schwierigkeiten, und zum anderen können sich höhere Mengen an Ölen und/oder Wachsen in kosmetischen Reinigungsmitteln nachteilig auf die Schaumeigenschaften - insbesondere die Schaummenge - der Mittel auswirken.
Ein weiteres Problem ist, dass angesichts der üblichen, eher kurzen Verweildauer eines Reinigungsmittels auf der Haut nur geringe Mengen an Pflegekomponenten auf der Haut abgeschieden werden können, bevor ein Großteil der Pflegekomponenten wieder abgespült wird.

Die Herstellung Öl-haltiger kosmetischer Reinigungsmittel wird in der Druckschrift US 2003/0108578 A1 beschrieben, jedoch sind die dort beschriebenen Kosmetika insbesondere hinsichtlich ihrer physikalischen Stabilität verbesserungswürdig.
Es besteht daher weiterhin der Bedarf nach pflegenden kosmetischen Reinigungsmitteln, die in physikalisch stabiler Form herstellbar sind, und die einen Konditionierungsvorteil für die Haut bieten.

Das Reinigungsmittel sollte eine effektive Menge mindestens eines Pflegeöls enthalten, ohne dass die Stabilisierung des Pflegeöls bei der Herstellung und/oder Lagerung des Reinigungsmittels zusätzliche, (energetisch) aufwendige Schritte erforderlich macht.

Die gereinigte Haut sollte sich nach der Anwendung der Mittel sauber, geschmeidig und glatt anfühlen. Ein weiteres Ziel der Erfindung war es pflegende kosmetische Reinigungsmittel herzustellen, die eine Lotion-ähnliche Optik und Haptik aufweisen.

Die zuvor angeführten Aufgaben lassen sich durch den Einsatz eines spezifischen anionischen Copolymers aus den Monomeren Ethylacrylat, Butylacrylat und Methacrylsäure in einem Fettstoffhaltigen kosmetischen Reinigungsmittel lösen. Sonnenschutzmittel, die neben weiteren Bestandteilen auch diese spezifischen Copolymere enthaltend, sind aus der internationalen Patentanmeldung WO 2010/046048 A1 bekannt.

Ein erster Gegenstand der Erfindung ist ein kosmetisches Reinigungsmittel mit einer Viskosität (Haake Rotationsviskosimeter RV 12, 20°C, Messeinrichtung MV; Spindel MV II; 8UPM) von 5000 bis 14000 mPas, enthaltend
a) mindestens ein anionisches Tensid
b) mindestens ein amphoteres Tensid
c) mindestens einen Fettstoff
d) mindestens ein anionisches Copolymer aus den Monomeren
   d1) Ethylacrylat
   d2) Butylacrylat
   d3) Methacrylsäure
   sowie gegebenenfalls weiteren Monomeren.

Die erfindungsgemäßen kosmetischen Reinigungsmittel zeichnen sich neben guten reinigenden und pflegenden Eigenschaften insbesondere durch eine Lotion-ähnliche Optik und Haptik einschließlich einer hohen Phasenstabilität und einer stabilen Viskosität aus. Die Viskosität (Haake Rotationsviskosimeter RV 12, 20°C, Messeinrichtung MV; Spindel MV II; 8UPM) bevorzugter kosmetischer Reinigungsmittel beträgt 6000 bis 13000 mPas und insbesondere 8000 bis 12000 mPas. Diese Viskositäten haben sich hinsichtlich der Gebrauchseigenschaften des Reinigungsmittels (Dosierung, Applikation) als auch hinsichtlich der erwünschten Lotion-ähnlichen Anmutung als besonders geeignet erwiesen.
Erfindungsgemäße kosmetische Reinigungsmittel enthalten als einen ersten wesentlichen Bestandteil mindestens ein anionisches Tensid a). Erfindungsgemäße kosmetische Reinigungsmittel enthalten, bezogen auf ihr Gesamtgewicht, vorzugsweise 0,1 bis 20 Gew.-%, bevorzugt 1,0 bis 15 Gew.-% und insbesondere 3,0 bis 12 Gew.-% anionisches Tensid a).

Zu den besonders geeigneten anionischen Tensiden zählen:
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare oder verzweigte, gesättigte oder ungesättigte Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und/oder -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- Alpha-Olefinsulfonate mit 8 bis 24 C-Atomen,
- Alkylsulfat- und/oder Alkylpolyglykolethersulfatsalze der Formel R-O(CH₂-CH₂O)ₓ-OSO₃⁻ X⁺, in der R eine bevorzugt lineare oder verzweigte, gesättigte oder ungesättigte Alkylgruppe mit 8 bis 30 C-Atomen, x = 0 oder 1 bis 12 und X ein Alkali- oder Ammoniumion ist,
- Sulfonate ungesättigter Fettsäuren mit 8 bis 24 C-Atomen und 1 bis 6 Doppelbindungen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel, 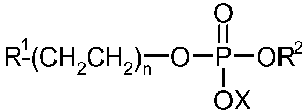 in der R¹ bevorzugt für einen aliphatischen Kohlenwasserstoffrest mit 8 bis 30 Kohlenstoffatomen, R² für Wasserstoff, einen Rest (CH₂CH₂O)ₙR¹ oder X, n für Zahlen von 0 bis 10 und X für Wasserstoff, ein Alkali- oder Erdalkalimetall oder NR³R⁴R⁵R⁶, mit R³ bis R⁶ unabhängig voneinander stehend für einen C₁ bis C₄ - Kohlenwasserstoffrest, steht.

Bevorzugte anionische Tenside sind Ethercarbonsäuren der zuvor genannten Formel, Acylsarcoside mit 8 bis 24 C-Atomen in der Acylgruppe, Sulfobernsteinsäuremono- und/oder - dialkylester mit 8 bis 24 C-Atomen in der Alkylgruppe und Sulfobernsteinsäuremono-alkylpolyoxy-ethylester mit 8 bis 24 C-Atomen in der Alkylgruppe und 1 bis 6 Oxyethylgruppen, Alpha-Olefinsulfonate mit 8 bis 24 C-Atomen und/oder Alkylsulfat- und/oder Alkylpolyglykolethersulfatsalze der zuvor genannten Formel.

Besonders bevorzugte anionische Tenside sind geradkettige oder verzweigte Alkylethersulfate, die einen Alkylrest mit 8 bis 18 und insbesondere mit 10 bis 16 C-Atomen sowie 1 bis 6 und insbesondere 2 bis 4 Ethylenoxideinheiten enthalten. Weiterhin besonders bevorzugte anionische Tenside sind geradkettige oder verzweigte Alkylsulfonate, die einen Alkylrest mit 8 bis 18 und insbesondere mit 10 bis 16 C-Atomen enthalten. Insbesondere bevorzugt sind die Natrium-, Magnesium und/oder Triethanolaminsalze linearer oder verzweigter Lauryl-, Tridecyl- und/oder Myristylsulfate, die einen Ethoxylierungsgrad von 2 bis 4 aufweisen. Kosmetische Reinigungsmittel, die mindestens ein anionisches Tensid a) aus der Gruppe der Alkylethersulfate, vorzugsweise der Alkylethersulfate die einen Alkylrest mit 8 bis 18, insbesondere mit 10 bis 16 C-Atomen sowie 1 bis 6, insbesondere 2 bis 4 Ethylenoxideinheiten aufweisen, werden erfindungsgemäß bevorzugt. Besonders bevorzugt werden anionische Tenside a) mit der INCI Bezeichnung Sodium Laureth Sulfate.

Ein zweiter wesentlicher Bestandteil erfindungsgemäßer kosmetischer Reinigungsmittel ist das amphotere Tensid b). Bevorzugte kosmetische Reinigungsmittel enthalten, bezogen auf ihr Gesamtgewicht, 0,01 bis 15 Gew.-%, vorzugsweise 0,075 bis 12 Gew.-% und insbesondere von 0,1 bis 8,0 Gew.-% amphoteres Tensid b).

Geeignete amphotere/zwitterionische Tenside können ausgewählt sein aus Verbindungen der folgenden Formeln (i) bis (v), in denen der Rest R jeweils für einen geradkettigen oder verzweigten, gesättigten oder ein- bzw. mehrfach ungesättigten Alkyl- oder Alkenlyrest mit 8 bis 24 Kohlenstoffatomen steht,

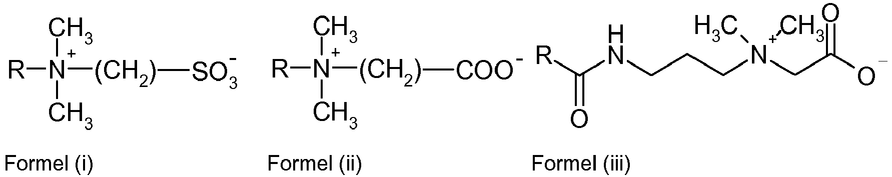

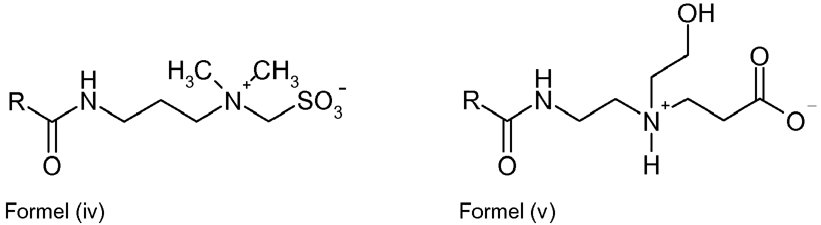

Besonders geeignete amphotere/zwitterionische Tenside sind Alkylamidoalkylbetaine und/oder Alkylampho(di)acetate der zuvor genannten Formeln (i) bis (v). Zu den insbesondere geeigneten amphoteren/zwitterionischen Tensiden zählen die unter der INCI-Bezeichnung bekannten Tenside Cocamidopropylbetaine und Disodium Cocoamphodiacetate. Kosmetische Reinigungsmittel, die mindestens ein amphoteres Tensid b) aus der Gruppe der Alkylamidoalkylbetaine und/oder Alkylampho(di)acetate, vorzugsweise aus der Gruppe der Tenside mit den INCI-Bezeichnungen Cocamidopropylbetaine und Disodium Cocoamphodiacetate enthalten, werden erfindungsgemäß bevorzugt.

Die Kombination anionischer und amphoterer Tenside hat sich insbesondere als vorteilhaft zur Erzielung optimaler Milde und zur Vermeidung bzw. Verringerung der Austrocknung der Haut während der Reinigung erwiesen.

Die kosmetischen Reinigungsmittel können in Ergänzung zu der Kombination aus anionischem Tensid a) und amphoteren Tensid b) zusätzlich ein nichtionisches Tensid enthalten. Geeignete nichtionische Tenside sind in den erfindungsgemäßen Mitteln bevorzugt in Mengen von 0 bis 12 Gew.-%, mehr bevorzugt von 0,25 bis 10 Gew.-%, besonders bevorzugt von 0,5 bis 8,0 Gew.-% und insbesondere von 1 bis 6,0 Gew.-% eingesetzt werden, wobei sich die Mengenangaben auf das Gesamtgewicht des kosmetischen Reinigungsmittels beziehen.
Zu den geeigneten nichtionischen Tensiden zählen
- C₈-C₃₀-Fettsäuremono- und -diestern von Anlagerungsprodukten von 1 bis 30 Mol Ethylenoxid an Glycerin,
- Anlagerungsprodukten von 2 bis 50 Mol Ethylenoxid und/oder 0 bis 5 Mol Propylenoxid an lineare und verzweigte Fettalkohole mit 8 bis 30 C-Atomen oder an Fettsäuren mit 8 bis 30 C-Atomen und an Alkylphenole mit 8 bis 15 C-Atomen in der Alkylgruppe,
- Sorbitanfettsäureestern und Anlagerungeprodukten von Ethylenoxid an Sorbitanfettsäure-ester,wie beispielsweise Polysorbate,
- Zuckerfettsäureestern und Anlagerungsprodukten von Ethylenoxid an Zuckerfettsäureester,
- Anlagerungsprodukten von Ethylenoxid an Fettsäurealkanolamide und Fettamine und/oder
- Alkylpolyglucosiden.
Besonders bevorzugt werden nichtionische Tenside aus der Gruppe der Anlagerungsprodukte von 2 bis 50 Mol Ethylenoxid und/oder 0 bis 5 Mol Propylenoxid an lineare und verzweigte Fettalkohole mit 8 bis 30 C-Atomen. Mehr bevorzugt sind C₁₂-C₂₀-Fettalkohole mit einem Ethoxylierungsgrad von 10 bis 25.

Der Gesamttensidgehalt bevorzugter kosmetischer Reinigungsmittel beträgt, bezogen auf das Gesamtgewicht des Reinigungsmittels, vorzugsweise maximal 16 Gew.-%, bevorzugt maximal 15 Gew.-%, besonders bevorzugt maximal 14 Gew.-% und insbesondere maximal 12,5 Gew.-%.

Als dritten wesentlichen Bestandteil enthalten die erfindungsgemäßen Mittel mindestens einen Fettstoff c). Der Gewichtsanteil des Fettstoffs am Gesamtgewicht des kosmetischen Reinigungsmittels beträgt vorzugsweise, 0,001 bis 10 Gew.-%, bevorzugt 0,01 bis 8,0 Gew.-% und insbesondere 0,1 bis 6,0 Gew.-%.

Bevorzugte Fettstoffe c) sind ausgewählt aus der Gruppe der Öle, der Wachse, der Fettsäuren, der Fettalkohole und der Esteröle, vorzugsweise aus der Gruppe der natürlichen Öle und der Esteröle enthält.

Geeignete Öle für die Herstellung der O/W-Emulsionen können ausgewählt sein aus mineralischen, natürlichen und synthetischen Ölkomponenten.
Als natürliche (pflanzliche) Öle können Triglyceride und Mischungen von Triglyceriden eingesetzt werden. Bevorzugte natürliche Öle sind Kokosnussöl, (süßes) Mandelöl, Walnussöl, Pfirsichkernöl, Aprikosenkernöl, Avocadoöl, Teebaumöl (Tea Tree Oil), Sojaöl, Sesamöl, Sonnenblumenöl, Tsubakiöl, Nachtkerzenöl, Reiskleieöl, Palmkernöl, Mangokernöl, Wiesenschaumkrautöl, Distelöl, Macadamianussöl, Traubenkernöl, Amaranthsamenöl, Arganöl, Bambusöl, Olivenöl, Weizenkeimöl, Kürbiskernöl, Malvenöl, Haselnussöl, Safloröl, Canolaöl, Sasanquaöl, Jojobaöl, Rambutanöl, Kakaoabutter und Shea-Butter.

Als mineralische Öle kommen insbesondere Mineralöle, Paraffin- und Isoparaffinöle sowie synthetische Kohlenwasserstoffe zum Einsatz. Ein Beispiel für einen einsetzbaren Kohlenwasserstoff ist beispielsweise das als Handelsprodukt erhältliche 1,3-Di-(2-ethylhexyl)-cyclohexan (Cetiol^{®} S).

Als synthetische Öle kommen Silikonverbindungen in Betracht. Geeignete Silikone können ausgewählt sein unter:
(i) Polyalkylsiloxanen, Polyarylsiloxanen, Polyalkylarylsiloxanen, die flüchtig oder nicht flüchtig, geradkettig, verzweigt oder cyclisch, vernetzt oder nicht vernetzt sind;
(ii) Polysiloxanen, die in ihrer allgemeinen Struktur eine oder mehrere organofunktionelle Gruppen enthalten, die ausgewählt sind unter:
   a) substituierten oder unsubstituierten aminierten Gruppen;
   b) (per)fluorierten Gruppen;
   c) Thiolgruppen;
   d) Carboxylatgruppen;
   e) hydroxylierten Gruppen;
   f) alkoxylierten Gruppen;
   g) Acyloxyalkylgruppen;
   h) amphoteren Gruppen;
   i) Bisulfitgruppen;
   j) Hydroxyacylaminogruppen;
   k) Carboxygruppen;
   l) Sulfonsäuregruppen; und
   m) Sulfat- oder Thiosulfatgruppen;
(iii) linearen Polysiloxan(A)- Polyoxyalkylen(B)- Blockcopoylmeren vom Typ (A-B)ₙ mit n > 3;
(iv) gepfropften Siliconpolymeren mit nicht siliconhaltigem, organischen Grundgerüst, die aus einer organischen Hauptkette bestehen, welche aus organischen Monomeren gebildet wird, die kein Silicon enthalten, auf die in der Kette sowie gegebenenfalls an mindestens einem Kettenende mindestens ein Polysiloxanmakromer gepfropft wurde;
(v) gepfropften Siliconpolymeren mit Polysiloxan- Grundgerüst, auf das nicht siliconhaltige, organische Monomere gepfropft wurden, die eine Polysiloxan-Hauptkette aufweisen, auf die in der Kette sowie gegebenenfalls an mindestens einem ihrer Enden mindestens ein organisches Makromer gepfropft wurde, das kein Silicon enthält;
(vi) oder deren Gemischen.

Als Ölkomponente kann weiterhin ein Dialkylether dienen. Einsetzbare Dialkylether sind insbesondere Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyln-dodecylether und n-Hexyl-n-undecylether sowie Di-tert.-butylether, Di-iso-pentylether, Di-3-ethyldecylether, tert.-Butyl-n-octylether, iso-Pentyl-n-octylether und 2-Methylpentyl-n-octylether. Besonders bevorzugt ist der Di-n-octylether, der im Handel unter der Bezeichnung Cetiol^{®} OE erhältlich ist.

Als Fettsäuren können lineare und/oder verzweigte, gesättigte und/oder ungesättigte Fettsäuren mit 6 - 30 Kohlenstoffatomen eingesetzt werden. Bevorzugt sind Fettsäuren mit 10 - 22 Kohlenstoffatomen. Hierunter wären beispielsweise zu nennen die Isostearinsäuren, wie die Handelsprodukte Emersol^{®} 871 und Emersol^{®} 875, und Isopalmitinsäuren wie das Handelsprodukt Edenor^{®} IP 95, sowie alle weiteren unter den Handelsbezeichnungen Edenor^{®} (Cognis) vertriebenen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen.
Besonders bevorzugt sind üblicherweise die Fettsäureschnitte, welche aus Cocosöl oder Palmöl erhältlich sind; insbesondere bevorzugt ist in der Regel der Einsatz von Stearinsäure.
Als Fettalkohole können eingesetzt werden gesättigte, ein- oder mehrfach ungesättigte, verzweigte oder unverzweigte Fettalkohole mit C₆ - C₃₀-, bevorzugt C₁₀ - C₂₂- und ganz besonders bevorzugt C₁₂ - C₂₂- Kohlenstoffatomen. Einsetzbar sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen. Solche Substanzen sind beispielsweise unter den Bezeichnungen Stenol^{®}, z.B. Stenol^{®} 1618 oder Lanette^{®}, z.B. Lanette^{®} O oder Lorol^{®}, z.B. Lorol^{®} C8, Lorol^{®} C14, Lorol^{®} C18, Lorol^{®} C8-18, HD-Ocenol^{®}, Crodacol^{®}, z.B. Crodacol^{®} CS, Novol^{®}, Eutanol^{®} G, Guerbitol^{®} 16, Guerbitol^{®} 18, Guerbitol^{®} 20, Isofol^{®} 12, Isofol^{®} 16, Isofol^{®} 24, Isofol^{®} 36, Isocarb^{®} 12, Isocarb^{®} 16 oder Isocarb^{®} 24 käuflich zu erwerben. Selbstverständlich können auch Wollwachsalkohole, wie sie beispielsweise unter den Bezeichnungen Corona^{®}, White Swan^{®}, Coronet^{®} oder Fluilan^{®} käuflich zu erwerben sind, eingesetzt werden.

Als natürliche oder synthetische Wachse können feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP eingesetzt werden.

Ein weiterer geeigneter Fettstoff sind die Esteröle. Unter Esterölen sind zu verstehen die Ester von C₆ - C₃₀ - Fettsäuren, insbesondere von C₅ - C₁₈ - Fettsäuren mit C₂ - C₃₀ - Fettalkoholen, insbesondere mit C₁₀ - C₂₂ - Fettalkoholen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen.
Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Besonders bevorzugt sind Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Cetyl Palmitate, Glycerintricaprylat, Kokosfettalkohol-caprinat/-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V).

Weitere geeignete Fettstoffe sind beispielsweise
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen,
- Glycerincarbonat oder Dicaprylylcarbonat (Cetiol^{®} CC),
- ethoxylierte oder nicht ethoxylierte Mono,- Di- und Trifettsäureester von gesättigten und/oder ungesättigten linearen und/oder verzweigten Fettsäuren mit Glycerin, wie beispielsweise die Handelsprodukte Tegin^{®} M, Monomuls^{®} 90-018, Monomuls^{®} 90-L12, Cetiol^{®} HE oder Cutina^{®} MD.

In einer bevorzugten Ausführungsform enthalten die erfindungsgemäßen kosmetischen Reinigungsmittel mindestens ein natürliches Öl und/oder mindestens einen der zuvor genannten Fettsäureester und/oder mindestens einen der zuvor genannten Glycerinmono- oder -diester und/oder mindestens einen der zuvor genannten Fettalkohole.

Zur Unterstützung der Abscheidung des pflegenden Fettstoffs auf der Haut kann es von Vorteil sein, wenn das kosmetische Reinigungsmittel weiterhin mindestens ein kationisches Abscheidungspolymer enthält. Die Anwesenheit mindestens eines kationischen Polymeren in dem kosmetischen Reinigungsmittel ist auch deshalb bevorzugt, weil kationische Polymere eine hautkonditionierende Wirkung aufweisen.

Geeignete kationische Polymere werden dem kosmetischen Reinigungsmittel, bezogen auf sein Gesamtgewicht, bevorzugt in einer Menge von 0,05 bis 10 Gew.-%, mehr bevorzugt von 0,1 bis 5 Gew.-% und insbesondere von 0,2 bis 3 Gew.-% zugesetzt.

Geeignete kationische Polymere sind beispielsweise:
- quaternisierte Cellulose-Derivate,
- hydrophob modifizierte Cellulosederivate,
- kationische Alkylpolyglycoside,
- kationiserter Honig,
- kationische Guar-Derivate,
- polymere Dimethyldiallylammoniumsalze und deren Copolymere mit Estern und Amiden von Acrylsäure und Methacrylsäure wie Poly(dimethyldiallylammoniumchlorid) oder (Dimethyldiallylammoniumchlorid-Acrylamid-Copolymer,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere,
- Vinylpyrrolidon-Vinylimidazoliummethochlorid-Copolymere,
- quaternierter Polyvinylalkohol,
sowie die unter den Bezeichnungen
- Polyquaternium 2, Polyquaternium 17, Polyquaternium 18, Polyquaternium-24, Polyquaternium 27, Polyquaternium-32, Polyquaternium-37, Polyquaternium 74 und Polyquaternium 89 bekannten Polymere.

Besonders bevorzugte kationische Polymere sind quaternisierte Cellulosepolymere, kationische Guarderivate und/oder kationische Polymere auf Acrylsäure(derivat)basis, die insbesondere ausgewählt sind aus den unter den INCI-Bezeichnungen bekannten Polymeren Guar Hydroxypropyltrimonium Chloride, Polyquaternium-6, Polyquaternium-7, Polyquaternium-10, Polyquaternium-37 und/oder Polyquaternium-67. Insbesondere bevorzugt ist das unter der INCI-Bezeichnung Polyquaternium-7 bekannte kationische Pflege- und Abscheidungspolymer.

Als vierten wesentlichen Bestandteil enthalten die erfindungsgemäßen kosmetischen Reinigungsmittel mindestens ein anionisches Copolymer d) aus den Monomeren d1) Ethylacrylat, d2) Butylacrylat, d3) Methacrylsäure sowie gegebenenfalls weiteren Monomeren. Der Gewichtsanteil des Copolymers d) am Gesamtgewicht des kosmetischen Reinigungsmittels beträgt vorzugsweise, 0,1 bis 10 Gew.-%, bevorzugt 0,5 bis 8,0 Gew.-% und insbesondere 1,0 bis 2,0 Gew.-%. Gegenüber vergleichbaren Polymeren zeichnen sich die anionischen Copolymere d) durch eine deutlich verbesserte physikalische Stabilität der kosmetischen Reinigungsmittel aus. Dies betrifft sowohl die Phasenstabilität als auch die Viskosität dieser Mittel. Das anionische Copolymer d) kann vernetzt oder unvernetzt sein. Bevorzugt werden jedoch vernetzte Copolymere d) eingesetzt.

In einer bevorzugten Ausführungsform beträgt der Gewichtsanteil der Monomere d1) Ethylacrylat, d2) Butylacrylat und d3) Methacrylsäure mindestens 80 Gew.-%, vorzugsweise mindestens 90 Gew.-%, bevorzugt mindestens 95 Gew.-% und insbesondere mindestens 98 Gew.-% am Gesamtgewicht der zur Herstellung des anionischen Copolymers d) eingesetzten Monomere. In einer besonders bevorzugten Ausführungsform wird ein anionisches Copolymer d) eingesetzt, welches ausschließlich auf den Monomeren d1) Ethylacrylat, d2) Butylacrylat, d3) Methacrylsäure sowie einem gegebenenfalls eingesetzten Vernetzungsmittel basiert.

Es wurde festgestellt, dass wässrige Emulsionen oder Dispersionen der zuvor genannten anionischen Copolymere d) bereits bei Raumtemperatur und pH-Werten im leicht aciden bis neutralen pH-Bereich Gelnetzwerke aus, durch die nicht nur höhere Mengen eines Fettstoffs über einen langen Zeitraum in dem Reinigungsmittel stabilisiert werden können, sondern durch die gleichzeitig die in dem Reinigungsmittel unlöslichen Pigmente, Perlglanz- und/oder Trübungsmittel stabil suspendiert bleiben.

Anorganische Pigment, Trübungs- und/oder Perlglanzmittel dienen der Erzielung eines optischen Lotioncharakters des Reinigungsmittels. Es ist bevorzugt, wenn der Gehalt an anorganischem(n) Pigment(en) und/oder Trübungs- und/oder Perlglanzmittel 0,01 bis 5 Gew.-%, bevorzugt 0,025 bis 3 Gew.-% und insbesondere 0,05 bis 2,5 Gew.-% beträgt, wobei sich die Mengenangaben auf das Gesamtgewicht des kosmetischen Reinigungsmittels beziehen.

Unter geeigneten anorganischen Pigmenten werden bevorzugt Pigmente verstanden, die einen Brechungsindex > 1,8, mehr bevorzugt > 1,9 und insbesondere > 2,0 aufweisen. Beispiele für besonders geeignete anorganische Pigmente sind die so genannten Weißpigmente wie Titandioxid, Lithopone, Zinkoxid, Zinksulfid und/oder Calciumcarbonat. Insbesondere geeignet ist Titandioxid, das in Verfahrensschritt a) besonders bevorzugt als 10-20%ige Dispersion in Wasser vorgelegt wird.

Zu den geeigneten Perlglanz- und/oder Trübungsmitteln zählen beispielsweise
- Mono- und/oder Diester des Ethylenglycols, 1,2-Propandiols, Glycerins und/oder eines Polyethylenglycols mit mindestens einer C₈-C₂₄-Carbonsäure, und/oder
- Styrol/Acrylat-Copolymere.
Besonders geeignet sind die unter den folgenden INCI-Bezeichnungen bekannten Trübungs- und/oder Perlglanzmittel: Glycol Distearate, Glycol Monostearate, PEG-3 Distearate, PEG-2 Distearate, Propylen Glycol Stearate und/oder Styrol/Acrylates-Copolymere. Insbesondere geeignet für die Verwendung in dem Herstellungsverfahren sind die unter den INCI-Bezeichnungen Glycol Distearate, Glycol Monostearate, PEG-3 Distearate und/oder Styrol/Acrylates-Copolymer bekannten Trübungs- und/oder Perlglanzmittel

Ein weiterer bevorzugter Inhaltsstoff der kosmetischen Reinigungsmittel sind Proteinhydrolysate, vorzugsweise pflanzlichen, tierischen oder marinen Ursprungs. Proteinhydrolysate werden in dem erfindungsgemäßen kosmetischen Reinigungsmittel bevorzugt in einer Menge von 0,01 bis 10 Gew.-%, mehr bevorzugt von 0,25 bis 7,5 Gew.-% und insbesondere in einer Menge von 0,05 bis 5 Gew.-% eingesetzt, jeweils bezogen auf das Gesamtgewicht des kosmetischen Reinigungsmittels.

Geeignete tierische Proteinhydrolysate sind beispielsweise Elastin-, Kollagen-, Keratin-, Seiden- und/oder Milcheiweiß-Proteinhydrolysate, die auch in Form von Salzen vorliegen können. Geeignete Proteinhydrolysate pflanzlichen Ursprungs sind beispielsweise Soja-, Mandel-, Reis-, Erbsen-, Kartoffel-, Raps- und/oder Weizenproteinhydrolysate.

Zu den geeigneten Proteinhydrolysaten marinen Ursprunges zählen beispielsweise Kollagenhydrolysate von Fischen oder Algen sowie Proteinhydrolysate von Muscheln bzw. Perlenhydrolysate.

Einsetzbar sind auch kationisierte Proteinhydrolysate, wobei das zugrunde liegende Proteinhydrolysat aus den zuvor beschriebenen tierischen, pflanzlichen und/oder marinen Quellen stammen kann.
Weiterhin sind unter kationischen Proteinhydrolysaten quaternierte Aminosäuren und deren Gemische zu verstehen. Die Quaternisierung der Proteinhydrolysate oder der Aminosäuren wird häufig mittels quarternärer Ammoniumsalze wie beispielsweise N,N-Dimethyl-N-(n-Alkyl)-N-(2-hydroxy-3-chloro-n-propyl)-ammoniumhalogeniden durchgeführt.
Weiterhin können die kationischen Proteinhydrolysate auch noch weiter derivatisiert sein.
Als typische Beispiele für geeignete kationische Proteinhydrolysate und/oder -derivate seien die unter den INCI - Bezeichnungen bekannten und im Handel erhältlichen Produkte genannt: Cocodimonium Hydroxypropyl Hydrolyzed Collagen, Cocodimopnium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Silk, Cocodimonium Hydroxypropyl Hydrolyzed Soy Protein, Cocodimonium Hydroxypropyl Hydrolyzed Wheat Protein, Cocodimonium Hydroxypropyl Silk Amino Acids, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed keratin, Hydroxypropyltrimonium Hydrolyzed Rice Bran Protein, Hydroxyproypltrimonium Hydrolyzed Silk, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Silk, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Silk, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Silk, Quaternium-79 Hydrolyzed Soy Protein, Quaternium-79 Hydrolyzed Wheat Protein.

Besonders bevorzugt für die Verwendung in dem erfindungsgemäßen Verfahren sind Proteinhydrolysate aus tierischen Quellen, insbesondere Elastin-, Kollagen-, Keratin- und/oder Seidenproteinhydrolysate, die bevorzugt ein mittleres Molgewicht (Gewichtsmittel) von 100 bis 2500, mehr bevorzugt von 200 bis 2000, besonders bevorzugt von 300 und 1500 und insbesondere von 400 bis 1200 Dalton aufweisen.

Die kosmetischen Reinigungsmittel können noch eine Reihe weiterer fakultativer Wirkstoffe enthalten, die auf der Haut vorteilhafte Eigenschaften bewirken können. Zu den bevorzugten fakultativen Wirkstoffen zählen beispielsweise:
- Vitamine, Vitaminderivate und/oder Vitaminvorstufen, die in dem kosmetischen Reinigungsmittel bevorzugt in einer Menge von 0,001 bis 10 Gew.-%, mehr bevorzugt von 0,005 bis 7,5 Gew.-% und insbesondere von 0,01 bis 5 Gew.-% enthalten sein können, und/oder
- Pflanzenextrakte und/oder Pflanzenmilche, die in dem kosmetischen Reinigungsmittel bevorzugt in einer Menge von 0,01 bis 10 Gew.-%, mehr bevorzugt von 0,025 bis 7,5 Gew.-%, besonders bevorzugt von 0,05 bis 5 Gew.-% und insbesondere von 0,075 bis 3 Gew.-% enthalten sein können.

Unter geeigneten Vitaminen sind bevorzugt die folgenden Vitamine, Provitamine und Vitaminvorstufen sowie deren Derivate zu verstehen: Vitamin A, Vitamin B, einschließlich Vitamin B₁ (Thiamin), Vitamin B₂ (Riboflavin) und Vitamin B₃ (Nicotinsäure und Nicotinsäureamid) sowie Vitamin B₅ (Pantothensäure und Panthenol) und Vitamin B₆ (Pyridoxin sowie Pyridoxamin und Pyridoxal), Vitamin C (Ascorbinsäure), Vitamin E (Tocopherole, insbesondere α-Tocopherol), Vitamin F, Vitamin H (Biotin). Bevorzugt ist die Verwendung der Vitamine, Provitamine und Vitaminvorstufen aus den Gruppen A, B, E und H. Insbesondere bevorzugt sind Nicotinsäureamid, Biotin, Pantolacton und/oder Panthenol.

Unter geeigneten Pflanzenextrakten und/oder Pflanzenmilchen sind Extrakte zu verstehen, die aus allen Teilen einer Pflanze hergestellt werden können. Geeignet sind vor allem die Extrakte aus Grünem Tee, Eichenrinde, Brennessel, Hamamelis, Hopfen, Kamille, Klettenwurzel, Schachtelhalm, Weißdorn, Lindenblüten, Litschi, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Ginseng, Ingwerwurzel, Echinacea purpurea, Olea europea, Boerhavia Diffusa-Wurzeln, Foeniculum vulgaris und Apim graveolens. Besonders bevorzugt für die Verwendung in den erfindungsgemäßen Reinigungsmitteln sind die Extrakte aus Grünem Tee, Brennessel, Hamamelis, Kamille, Aloe Vera, Ginseng, Echinacea purpurea, Olea europea und/oder Boerhavia Diffusa-Wurzeln.

Weitere Wirk-, Hilfs- und Zusatzstoffe, die in dem erfindungsgemäßen Verfahren eingesetzt werden können, sind beispielsweise:
- Feuchthaltemittel,
- Parfums,
- UV-Filter,
- Weitere Verdickungsmittel wie Gelatine oder Pflanzengumme, beispielsweise Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone und Schichtsilikate wie z. B. Bentonit oder vollsynthetische Hydrokolloide wie z. B. Polyvinylalkohol, die Ca-, Mg- oder Zn - Seifen,
- Strukturanten wie Maleinsäure und Milchsäure,
- Dimethylisosorbid,
- Cyclodextrine,
- faserstrukturverbessernde Wirkstoffe, insbesondere Mono-, Di- und Oligosaccharide wie beispielsweise Glucose, Galactose, Fructose, Fruchtzucker und Lactose,
- Farbstoffe zum Anfärben des Mittels,
- Substanzen zur Einstellung des pH-Wertes, beispielsweise α- und β-Hydroxycarbonsäuren wie Citronensäure, Milchsäure, Äpfelsäure, Glycolsäure,
- Wirkstoffe wie Bisabolol,
- Komplexbildner wie EDTA, NTA, β-Alanindiessigsäure und Phosphonsäuren,
- Ceramide. Unter Ceramiden werden N-Acylsphingosin (Fettsäureamide des Sphingosins) oder synthetische Analogen solcher Lipide (sogenannte Pseudo-Ceramide) verstanden,
- Treibmittel wie Propan-Butan-Gemische, N₂O, Dimethylether, CO₂ und Luft,
- Antioxidantien,
- Konsistenzgeber wie Zuckerester, Polyolester oder Polyolalkylether,
- Konservierungsmittel, wie beispielsweise Natriumbenzoat und/oder Salicylsäure,
- Viskositätsregler wie Salze (NaCl).

Die kosmetischen Reinigungsmittel umfassen vorzugsweise weiterhin einen kosmetisch akzeptablen Träger. Unter einem kosmetisch akzeptablen Träger wird bevorzugt ein wässriger oder wässrig-alkoholischer Träger verstanden. Bevorzugt umfasst das kosmetische Mittel bezogen auf sein Gesamtgewicht mindestens 40 Gew.-%, vorzugsweise 50 bis 85 Gew.-% und insbesondere 60 bis 80 Gew.-% Wasser.
Weiterhin kann das kosmetische Mittel 0,01 bis 40 Gew.-%, bevorzugt 0,05 bis 35 Gew.-% und insbesondere 0,1 bis 30 Gew.-% mindestens eines Alkohols enthalten, der ausgewählt sein kann aus Ethanol, Ethyldiglykol, 1-Propanol, 2-Propanol, Isopropanol, 1,2-Propylenglycol, Glycerin, 1-Butanol, 2-Butanol, 1,2-Butandiol, 1,3-Butandiol, 1-Pentanol, 2-Pentanol, 1,2-Pentandiol, 1,5-Pentandiol, 1, Hexanol, 2-Hexanol, 1,2-Hexandiol, 1,6-Hexandiol, Sorbitol, Benzylalkohol, Phenoxyethanol oder Mischungen dieser Alkohole. Bevorzugt sind die wasserlöslichen Alkohole. Insbesondere bevorzugt sind Ethanol, Ethyldiglykol, 1-Propanol, 2-Propanol, Isopropanol, 1,2-Propylenglycol, Glycerin, Benzylalkohol und/oder Phenoxyethanol sowie Mischungen dieser Alkohole.

Die erfindungsgemäßen kosmetischen Mittel weisen bevorzugt einen pH-Wert im Bereich von 3 bis 7, mehr bevorzugt von 4 bis 6,5, besonders bevorzugt von 5 bis 6,25 und insbesondere von 5,5 bis 6 auf.

Die Zusammensetzung einiger bevorzugter Reinigungsmittel kann den folgenden Tabellen entnommen werden (Angaben in Gew.-% bezogen auf das Gesamtgewicht des Reinigungsmittels sofern nicht anders angegeben).

| | Formel 1 | Formel 2 | Formel 3 | Formel 4 | Formel 5 |
|---|---|---|---|---|---|
| Anionisches Tensid a) | 0,1 bis 20 | 1,0 bis 15 | 1,0 bis 15 | 3,0 bis 12 | 3,0 bis 12 |
| Amphoteres Tensid b) | 0,01 bis 15 | 0,01 bis 15 | 0,1 bis 8,0 | 0,1 bis 8,0 | 0,1 bis 8,0 |
| Fettstoff c) | 0,001 bis 10 | 0,01 bis 8,0 | 0,01 bis 8,0 | 0,1 bis 6,0 | 0,1 bis 6,0 |
| Anionisches Copolymer d) | 0,1 bis 10 | 0,1 bis 10 | 0,5 bis 8,0 | 0,5 bis 8,0 | 1,0 bis 2,0 |
| Misc | add 100 | add 100 | add 100 | add 100 | add 100 |
| | | | | | |

| | Formel 6 | Formel 7 | Formel 8 | Formel 9 | Formel 10 |
|---|---|---|---|---|---|
| Sodium Laureth Sulfate | 0,1 bis 20 | 1,0 bis 15 | 1,0 bis 15 | 3,0 bis 12 | 3,0 bis 12 |
| Amphoteres Tensid b) | 0,01 bis 15 | 0,01 bis 15 | 0,1 bis 8,0 | 0,1 bis 8,0 | 0,1 bis 8,0 |
| Fettstoff c) | 0,001 bis 10 | 0,01 bis 8,0 | 0,01 bis 8,0 | 0,1 bis 6,0 | 0,1 bis 6,0 |
| Anionisches Copolymer d) | 0,1 bis 10 | 0,1 bis 10 | 0,5 bis 8,0 | 0,5 bis 8,0 | 1,0 bis 2,0 |
| Misc | add 100 | add 100 | add 100 | add 100 | add 100 |
| | | | | | |

| | Formel 11 | Formel12 | Formel 13 | Formel 14 | Formel 15 |
|---|---|---|---|---|---|
| Anionisches Tensid a) | 0,1 bis 20 | 1,0 bis 15 | 1,0 bis 15 | 3,0 bis 12 | 3,0 bis 12 |
| Cocoamidopropyl betaine | 0,01 bis 15 | 0,01 bis 15 | 0,1 bis 8,0 | 0,1 bis 8,0 | 0,1 bis 8,0 |
| Fettstoff c) | 0,001 bis 10 | 0,01 bis 8,0 | 0,01 bis 8,0 | 0,1 bis 6,0 | 0,1 bis 6,0 |
| Anionisches Copolymer d) | 0,1 bis 10 | 0,1 bis 10 | 0,5 bis 8,0 | 0,5 bis 8,0 | 1,0 bis 2,0 |
| Misc | add 100 | add 100 | add 100 | add 100 | add 100 |

| | Formel 16 | Formel 17 | Formel 18 | Formel 19 | Formel 20 |
|---|---|---|---|---|---|
| Anionisches Tensid a) | 0,1 bis 20 | 1,0 bis 15 | 1,0 bis 15 | 3,0 bis 12 | 3,0 bis 12 |
| Amphoteres Tensid b) | 0,01 bis 15 | 0,01 bis 15 | 0,1 bis 8,0 | 0,1 bis 8,0 | 0,1 bis 8,0 |
| Esteröl | 0,001 bis 10 | 0,01 bis 8,0 | 0,01 bis 8,0 | 0,1 bis 6,0 | 0,1 bis 6,0 |
| Anionisches Copolymer d) | 0,1 bis 10 | 0,1 bis 10 | 0,5 bis 8,0 | 0,5 bis 8,0 | 1,0 bis 2,0 |
| Misc | add 100 | add 100 | add 100 | add 100 | add 100 |
| | | | | | |

| | Formel 21 | Formel 22 | Formel 23 | Formel 24 | Formel 25 |
|---|---|---|---|---|---|
| Sodium Laureth Sulfate | 0,1 bis 20 | 1,0 bis 15 | 1,0 bis 15 | 3,0 bis 12 | 3,0 bis 12 |
| Cocoamidopropyl betaine | 0,01 bis 15 | 0,01 bis 15 | 0,1 bis 8,0 | 0,1 bis 8,0 | 0,1 bis 8,0 |
| Fettstoff | 0,001 bis 10 | 0,01 bis 8,0 | 0,01 bis 8,0 | 0,1 bis 6,0 | 0,1 bis 6,0 |
| Anionisches Copolymer * | 0,1 bis 10 | 0,1 bis 10 | 0,5 bis 8,0 | 0,5 bis 8,0 | 1,0 bis 2,0 |
| Misc | add 100 | add 100 | add 100 | add 100 | add 100 |
| * quervernetztes Copolymer aus den Monomeren Ethylacrylat, Butylacrylat und Methacrylsäure | | | | | |

| | Formel 26 | Formel 27 | Formel 28 | Formel 29 | Formel 30 |
|---|---|---|---|---|---|
| Anionisches Tensid a) | 0,1 bis 20 | 1,0 bis 15 | 1,0 bis 15 | 3,0 bis 12 | 3,0 bis 12 |
| Amphoteres Tensid b) | 0,01 bis 15 | 0,01 bis 15 | 0,1 bis 8,0 | 0,1 bis 8,0 | 0,1 bis 8,0 |
| Fettstoff c) | 0,001 bis 10 | 0,01 bis 8,0 | 0,01 bis 8,0 | 0,1 bis 6,0 | 0,1 bis 6,0 |
| Anionisches Copolymer d) | 0,1 bis 10 | 0,1 bis 10 | 0,5 bis 8,0 | 0,5 bis 8,0 | 1,0 bis 2,0 |
| Wasser | 50 bis 85 | 50 bis 85 | 50 bis 85 | 60 bis 80 | 60 bis 80 |
| Misc | add 100 | add 100 | add 100 | add 100 | add 100 |
| | | | | | |

| | Formel31 | Formel 32 | Formel 33 | Formel 34 | Formel 35 |
|---|---|---|---|---|---|
| Sodium Laureth Sulfate | 0,1 bis 20 | 1,0 bis 15 | 1,0 bis 15 | 3,0 bis 12 | 3,0 bis 12 |
| Cocoamidopropyl betaine | 0,01 bis 15 | 0,01 bis 15 | 0,1 bis 8,0 | 0,1 bis 8,0 | 0,1 bis 8,0 |
| Fettstoff | 0,001 bis 10 | 0,01 bis 8,0 | 0,01 bis 8,0 | 0,1 bis 6,0 | 0,1 bis 6,0 |
| Anionisches Copolymer * | 0,1 bis 10 | 0,1 bis 10 | 0,5 bis 8,0 | 0,5 bis 8,0 | 1,0 bis 2,0 |
| Wasser | 50 bis 85 | 50 bis 85 | 50 bis 85 | 60 bis 80 | 60 bis 80 |
| Misc | add 100 | add 100 | add 100 | add 100 | add 100 |
| * quervernetztes Copolymer aus den Monomeren Ethylacrylat, Butylacrylat und Methacrylsäure | | | | | |

| | Formel 36 | Formel 37 | Formel 38 | Formel 39 | Formel 40 |
|---|---|---|---|---|---|
| Sodium Laureth Sulfate | 0,1 bis 20 | 1,0 bis 15 | 1,0 bis 15 | 3,0 bis 12 | 3,0 bis 12 |
| Cocoamidopropyl betaine | 0,01 bis 15 | 0,01 bis 15 | 0,1 bis 8,0 | 0,1 bis 8,0 | 0,1 bis 8,0 |
| Fettstoff | 0,001 bis 10 | 0,01 bis 8,0 | 0,01 bis 8,0 | 0,1 bis 6,0 | 0,1 bis 6,0 |
| Anionisches Copolymer * | 0,1 bis 10 | 0,1 bis 10 | 0,5 bis 8,0 | 0,5 bis 8,0 | 1,0 bis 2,0 |
| Perlglanz und/oder | 0,01 bis 5,0 | 0,025 bis | 0,025 bis | 0,05 bis 2,5 | 0,05 bis |
| Trübungsmittel | | 3,0 | 3,0 | | 2,5 |
| Wasser | 50 bis 85 | 50 bis 85 | 50 bis 85 | 60 bis 80 | 60 bis 80 |
| Misc | add 100 | add 100 | add 100 | add 100 | add 100 |
| * quervernetztes Copolymer aus den Monomeren Ethylacrylat, Butylacrylat und Methacrylsäure | | | | | |

| | Formel41 | Formel 42 | Formel 43 | Formel 44 | Formel 45 |
|---|---|---|---|---|---|
| Sodium Laureth Sulfate | 0,1 bis 20 | 1,0 bis 15 | 1,0 bis 15 | 3,0 bis 12 | 3,0 bis 12 |
| Cocoamidopropyl betaine | 0,01 bis 15 | 0,01 bis 15 | 0,1 bis 8,0 | 0,1 bis 8,0 | 0,1 bis 8,0 |
| Fettstoff | 0,001 bis 10 | 0,01 bis 8,0 | 0,01 bis 8,0 | 0,1 bis 6,0 | 0,1 bis 6,0 |
| Anionisches Copolymer * | 0,1 bis 10 | 0,1 bis 10 | 0,5 bis 8,0 | 0,5 bis 8,0 | 1,0 bis 2,0 |
| Kationisches Polymer | 0,05 bis 10 | 0,1 bis 5,0 | 0,1 bis 5,0 | 0,2 bis 3,0 | 0,2 bis 3,0 |
| Wasser | 50 bis 85 | 50 bis 85 | 50 bis 85 | 60 bis 80 | 60 bis 80 |
| Misc | add 100 | add 100 | add 100 | add 100 | add 100 |
| * quervernetztes Copolymer aus den Monomeren Ethylacrylat, Butylacrylat und Methacrylsäure | | | | | |

| | Formel 46 | Formel 47 | Formel 48 | Formel 49 | Formel 50 |
|---|---|---|---|---|---|
| Sodium Laureth Sulfate | 0,1 bis 20 | 1,0 bis 15 | 1,0 bis 15 | 3,0 bis 12 | 3,0 bis 12 |
| Cocoamidopropyl betaine | 0,01 bis 15 | 0,01 bis 15 | 0,1 bis 8,0 | 0,1 bis 8,0 | 0,1 bis 8,0 |
| Fettstoff | 0,001 bis 10 | 0,01 bis 8,0 | 0,01 bis 8,0 | 0,1 bis 6,0 | 0,1 bis 6,0 |
| Anionisches Copolymer * | 0,1 bis 10 | 0,1 bis 10 | 0,5 bis 8,0 | 0,5 bis 8,0 | 1,0 bis 2,0 |
| Perlglanz und/oder Trübungsmittel | 0,01 bis 5,0 | 0,025 bis 3,0 | 0,025 bis 3,0 | 0,05 bis 2,5 | 0,05 bis 2,5 |
| Kationisches Polymer | 0,05 bis 10 | 0,1 bis 5,0 | 0,1 bis 5,0 | 0,2 bis 3,0 | 0,2 bis 3,0 |
| Wasser | 50 bis 85 | 50 bis 85 | 50 bis 85 | 60 bis 80 | 60 bis 80 |
| Misc | add 100 | add 100 | add 100 | add 100 | add 100 |
| * quervernetztes Copolymer aus den Monomeren Ethylacrylat, Butylacrylat und Methacrylsäure | | | | | |

Wie eingangs ausgeführt zeichnen sich die erfindungsgemäßen kosmetischen Mittel neben ihren reinigenden Eigenschaften insbesondere auch durch eine gute Pflegewirkung aus. Ein weiterer Gegenstand der vorliegenden Anmeldung ist die Verwendung eines erfindungsgemäßen kosmetischen Reinigungsmittels zur pflegenden Hautreinigung.

Die Herstellung erfindungsgemäßer kosmetischer Reinigungsmittel erfolgt vorzugsweise in einem mehrstufigen Verfahren, insbesondere unter Einarbeitung des in diesen Mitteln enthaltenen Fettstoffs in Form einer O/W-Emulsion. Ein letzter Gegenstand der vorliegenden Anmeldung ist daher ein Verfahren zur Herstellung eines kosmetischen Reinigungsmittels mit einer Viskosität (Haake Rotationsviskosimeter RV 12, 20°C, Meßeinrichtung MV; Spindel MV II; 8UPM) von 5000 bis 14000 mPas, das die folgenden Schritte aufweist:
a) Bereitstellen einer Zubereitung, die mindestens anionisches Copolymer aus den Monomeren
   d1) Ethylacrylat
   d2) Butylacrylat
   d3) Methacrylsäure
   sowie gegebenenfalls weiteren Monomeren enthält,
b) Bereitstellen einer O/W-Emulsion mit mindestens einem Fettstoff,
c) Vermischen der O/W-Emulsion aus Verfahrensschritt b) mit der Zubereitung aus Verfahrensschritt a),
d) Bereitstellen einer vorzugsweise wässrigen Zubereitung, die mindestens ein anionisches Tensid und mindestens ein amphoteres Tensid enthält,
e) Vermischen der Zubereitung aus Verfahrensschritt d) mit der Mischung aus Verfahrensschritt c).

In einer bevorzugten Ausführungsform des erfindungsgemäßen Verfahrens wird die Zubereitung aus Verfahrensschritt a) vor dem Vermischen mit der O/W Emulsion aus Verfahrensschritt b) mit einer wässrigen Dispersion, die mindestens ein anorganisches Pigment und/oder mindestens ein Trübungs- und/oder Perlglanzmittel enthält, vermischt.

## Patentansprüche

1. Kosmetisches Reinigungsmittel mit einer Viskosität (Haake Rotationsviskosimeter RV 12, 20°C, Meßeinrichtung MV; Spindel MV II; 8UPM) von 5000 bis 14000 mPas, enthaltend
a) mindestens ein anionisches Tensid
b mindestens ein amphoteres Tensid
c) mindestens einen Fettstoff
d) mindestens ein anionisches Copolymer aus den Monomeren
d1) Ethylacrylat
d2) Butylacrylat
d3) Methacrylsäure
sowie gegebenenfalls weiteren Monomeren.

2. Kosmetisches Reinigungsmittel nach einem der vorherigen Ansprüche, **dadurch gekennzeichnet, dass** das kosmetische Reinigungsmittel, bezogen auf sein Gesamtgewicht, 0,1 bis 20 Gew.-%, vorzugsweise 1,0 bis 15 Gew.-% und insbesondere 3,0 bis 12 Gew.-% anionisches Tensid a) enthält.

3. Kosmetisches Reinigungsmittel nach einem der vorherigen Ansprüche, **dadurch gekennzeichnet, dass** das kosmetische Reinigungsmittel mindestens ein anionisches Tensid a) aus der Gruppe der Alkylethersulfate, vorzugsweise der Alkylethersulfate die einen Alkylrest mit 8 bis 18, insbesondere mit 10 bis 16 C-Atomen sowie 1 bis 6, insbesondere 2 bis 4 Ethylenoxideinheiten aufweisen, enthält.

4. Kosmetisches Reinigungsmittel nach einem der vorherigen Ansprüche, **dadurch gekennzeichnet, dass** das kosmetische Reinigungsmittel, bezogen auf sein Gesamtgewicht, 0,01 bis 15 Gew.-%, vorzugsweise 0,075 bis 12 Gew.-% und insbesondere von 0,1 bis 8,0 Gew.-% amphoteres Tensid b) enthält.

5. Kosmetisches Reinigungsmittel nach einem der vorherigen Ansprüche, **dadurch gekennzeichnet, dass** das kosmetische Reinigungsmittel mindestens ein amphoteres Tensid b) aus der Gruppe der Alkylamidoalkylbetaine und/oder Alkylampho(di)acetate, vorzugsweise aus der Gruppe der Tenside mit den INCI-Bezeichnungen Cocamidopropylbetaine und Disodium Cocoamphodiacetate enthält.

6. Kosmetisches Reinigungsmittel nach einem der vorherigen Ansprüche, **dadurch gekennzeichnet, dass** das kosmetische Reinigungsmittel, bezogen auf sein Gesamtgewicht, 0,001 bis 10 Gew.-%, vorzugsweise 0,01 bis 8,0 Gew.-% und insbesondere 0,1 bis 6,0 Gew.-% mindestens eines Fettstoffs c) enthält.

7. Kosmetisches Reinigungsmittel nach einem der vorherigen Ansprüche, **dadurch gekennzeichnet, dass** das kosmetische Reinigungsmittel mindestens einen Fettstoff c) aus der Gruppe der Öle, der Wachse, der Fettsäuren, der Fettalkohole und der Esteröle, vorzugsweise aus der Gruppe der natürlichen Öle und der Esteröle enthält.

8. Kosmetisches Reinigungsmittel nach einem der vorherigen Ansprüche, **dadurch gekennzeichnet, dass** das kosmetische Reinigungsmittel, bezogen auf sein Gesamtgewicht, 0,1 bis 10 Gew.-%, vorzugsweise 0,5 bis 8,0 Gew.-% und insbesondere 1,0 bis 2,0 Gew.-% des anionischen Copolymers d) enthält.

9. Verwendung eines kosmetisches Reinigungsmittels mit einer Viskosität (Haake Rotationsviskosimeter RV 12, 20°C, Meßeinrichtung MV; Spindel MV II; 8UPM) von 5000 bis 14000 mPas, enthaltend
a) mindestens ein anionisches Tensid
b mindestens ein amphoteres Tensid
c) mindestens einen Fettstoff
d) mindestens ein anionisches Copolymer aus den Monomeren
d1) Ethylacrylat
d2) Butylacrylat
d3) Methacrylsäure
sowie gegebenenfalls weiteren Monomeren
zur pflegenden Hautreinigung.

10. Verfahren zur Herstellung eines kosmetischen Reinigungsmittels mit einer Viskosität (Haake Rotationsviskosimeter RV 12, 20°C, Meßeinrichtung MV; Spindel MV II; 8UPM) von 5000 bis 14000 mPas, das die folgenden Schritte aufweist:
a) Bereitstellen einer Zubereitung, die mindestens anionisches Copolymer aus den Monomeren
d1) Ethylacrylat
d2) Butylacrylat
d3) Methacrylsäure
sowie gegebenenfalls weiteren Monomeren enthält,
b) Bereitstellen einer O/W-Emulsion mit mindestens einem Fettstoff,
c) Vermischen der O/W-Emulsion aus Verfahrensschritt b) mit der Zubereitung aus Verfahrensschritt a),
d) Bereitstellen einer vorzugsweise wässrigen Zubereitung, die mindestens ein anionisches Tensid und mindestens ein amphoteres Tensid enthält,
e) Vermischen der Zubereitung aus Verfahrensschritt d) mit der Mischung aus Verfahrensschritt c).
